# EUROPEAN PATENT APPLICATION

(11) **EP 2 363 028 A1**
(43) Date of publication of application: **07.09.2011**
(21) Application number: 11003365.1
(22) Date of filing: 18.10.2006
(51) Int. Cl.: A23L 1/29

(54) **High-calorie nutritional supplement**

(30) Priority: 23.03.2006 US 785070 P
(62) Divisional of application: 06826146.0
(71) Applicant: Nestec S.A., 1800 Vevey (CH); Novartis Pharma GmbH, 1230 Wien (AT)
(72) Inventor: Olson, Erin, Plymouth, Minnesota 55442 (US); Tisdale, Michael J., Warwickshire, Warwickshire, CV35 8LW (US); Greenberg, Norman Alan, New Hope, Minnesota 55427 (US)
(74) Representative: Lock, Graham James

(57) **Abstract**

The invention provides a nutritional supplement comprising a protein source including milk protein isolate and/or canola plant protein, as well as related methods for its production and use in treating a nutritional deficiency in an individual.

## Description

### BACKGROUND OF THE INVENTION

### 1. Technical Field

The invention relates generally to nutritional supplements, and more particularly, to a high-calorie nutritional supplement comprising milk protein isolate as a primary or sole protein source. The invention further relates to methods of manufacturing and administering such a nutritional supplement.

### 2. Background Art

An individual's caloric intake is derived from three general sources: proteins, fats, and carbohydrates. The proper proportion of each calorie source in an individual's diet depends on a number of factors, including, for example, the individual's age, level of physical activity, and any diseases, disorders, or conditions the individual may be suffering from. For example, elderly individuals generally require fewer total calories and fewer calories from fat than do younger individuals.

Nutritional supplements have been developed to increase the total calories consumed and/or alter the proportion of proteins, fats, and carbohydrates in an individual's diet. Individuals who are ill may experience decreased appetite and therefore benefit from a nutritional supplement with a high caloric content capable of providing sufficient total calories in a reduced volume.

Individuals suffering from weight loss, in particular, may benefit from high-calorie nutritional supplements. Diseases, disorders, and conditions characterized by weight loss or with which weight loss is commonly associated include, for example, anorexia nervosa; wasting diseases, including cancer, acquired immune deficiency syndrome (AIDS), and sarcopenia (age-related loss of muscle mass); chronic illness; functional limitations, including psychological disorders and physical incapacitation; diminished cognitive ability; and wounds.

Many cases of unintentional weight loss involve the loss of muscle mass. In such cases, nutritional supplements containing a greater proportion of proteins and/or amino acids may be particularly beneficial, providing raw materials for the production of new muscle tissue. However, increasing both the total caloric content and protein content of a nutritional supplement tends to increase the supplement's viscosity. This can be problematic in both orally- and enterally-administrable supplements. In addition, supplements having a high protein content also tend to exhibit an objectionable flavor, posing an additional problem for orally-administrable supplements.

Milk protein provides a high concentration of digestible amino acids and is therefore ideally suited for inclusion as a protein source in nutritional supplements. More specifically, milk protein provides both slow-absorbed (casein) and fast-absorbed (whey) proteins. The slowly-absorbed casein protein component promotes post-prandial protein deposition by an inhibition of protein breakdown without excessive increases in amino acid concentrations. The rapidly-absorbed whey protein component stimulates protein synthesis. Thus, adjustment of the proportion of casein and whey proteins allows for the tailoring of protein content to meet the needs of a particular individual. For example, the inclusion of a greater proportion of whey protein will promote the production of muscle tissue, particularly advantageous in an individual suffering from a loss of muscle tissue.

As explained above, increased protein content tends to increase the viscosity of a nutritional supplement. This is particularly true of milk proteins, soy protein, and caseinates. Attempts to reduce the supplement's viscosity, including kettle heating and hold times, high temperature short time [HTST] processing, and sterility processing such direct steam injection (DSI), and retort ultra-high temperature (UHT) alone, have failed to adequately reduce supplement viscosity and have resulted in undesirable results, such as burning of proteins, increased viscosity, undesirable mouth feel such a chalkiness, browning, product separation, and padding. As a result, protein sources other than milk protein, such as soy protein and caseinates, have been used in high-protein nutritional supplements and the caloric content has been limited. The uses of such protein sources are not, however, without significant drawbacks. For example, as compared to milk protein, soy protein is deficient in methionine, lysine, and the branched chain amino acids (leucine, isoleucine, and valine). In addition, the presence of trypsin inhibitors in soy protein affects its digestibility, and, therefore, its bioavailability. As a result, soy protein is generally considered to be a lower-quality protein source than milk protein.

Canola proteins have been used for animal feed and not human consumption because there is no history of consumption. Canola was previously known as rape seed. Rape seed is high in erucic acid, a toxin for monogastric mammals. The erucic acid has been bred out of the plant which has lead to a large annual planting of canola (derived from the phrase, Canadian oil). Without a history of human consumption, the left over plant material after canola oil production has gone to animal feed. Companies have recently established that the amino acid profile of the canola seed proteins is of excellent quality.

Accordingly, there is a need in the art for a high-calorie nutritional supplement comprising a high-quality protein source that does not suffer from the deficiencies above.

### SUMMARY OF THE INVENTION

The invention provides a nutritional supplement comprising a protein source including milk protein isolate and/or canola plant proteins, as well as related methods for its production and use in treating a nutritional deficiency in an individual.

In a first aspect, the invention provides a nutritional supplement comprising: a protein source including milk protein isolate and/or canola plant proteins; a fat source; and a carbohydrate source, wherein the nutritional supplement has a caloric content of between about 2.25 and about 3.25 calories per milliliter and has a viscosity of less than about 120 centipoises.

In a second aspect, the invention provides a method of producing a liquid nutritional supplement, the method comprising: providing a mixture including water and a protein source including milk protein isolate and/or canola plant proteins; subjecting the mixture to direct steam injection (DSI); and homogenizing the mixture, wherein the liquid nutritional supplement has a viscosity of less than about 120 centipoises.

In a third aspect, the invention provides a method of treating a nutritional deficiency in an individual, the-method comprising: administering to the individual a nutritional supplement including: a protein source including milk protein isolate and/or canola plant protein; a fat source; and a carbohydrate source, wherein the nutritional supplement has a caloric content of between about 2.25 and about 3.25 calories per milliliter and has a viscosity of less than about 100 centipoises.

The illustrative aspects of the present invention are designed to solve the problems herein described and other problems not discussed, which are discoverable by a skilled artisan.

### BRIEF DESCRIPTION OF THE DRAWING

These and other features of this invention will be more readily understood from the following detailed description of the various aspects of the invention taken in conjunction with the accompanying drawing that depicts various embodiments of the invention, in which:

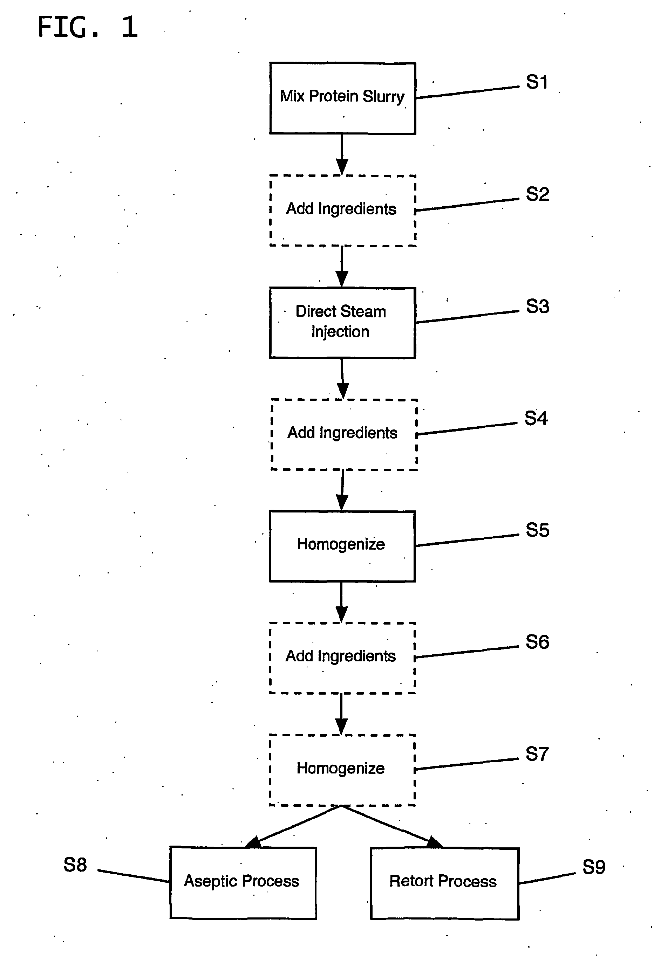

FIG. 1 shows a flow chart of an illustrative method according to the invention.

It is noted that the drawing is not to scale. The drawing is intended to depict only typical aspects of the invention, and therefore should not be considered as limiting the scope of the invention.

### DETAILED DESCRIPTION

As indicated above, the invention provides a high-calorie nutritional supplement comprising milk protein isolate as a primary or sole protein source. The invention further provides methods of manufacturing and administering such a nutritional supplement.

As used herein, the terms "treatment" and "treat" refer to both prophylactic or preventive treatment and curative or disease-modifying treatment, including treatment of patients at risk of contracting a disease or suspected to have contracted a disease, as well as patients who are ill or have been diagnosed as suffering from a disease or medical condition. The terms "treatment" and "treat" also refer to the maintenance and/or promotion of health in an individual not suffering from a disease but who may be susceptible to the development of an unhealthy condition, such as nitrogen imbalance, muscle loss, or weight loss. Consequently, an "effective amount" is an amount that treats a disease or medical condition in an individual or, more generally; provides a nutritional, physiological, or medical benefit to the individual. In addition, while the terms "individual" and "patient" are often used herein to refer to a human, the invention is not so limited. Accordingly, the terms "individual" and "patient" refer to any mammal suffering from or at risk for a medical condition, such as weight loss.

### Direct Steam injection (DSI) of Milk Protein Isolate

Surprisingly, it has been found that processing milk protein isolate using direct steam injection (DSI) reduces the viscosity of milk protein isolate to a level suitable for use in a nutritional supplement, particularly an orally-administrable nutritional supplement. DSI has been used in the food industry since the early 1930s. As such, its principles would be known to one skilled in the art and will not, therefore, be detailed herein.

Turning now to the figure, FIG. 1 shows a flow chart of a method for producing a liquid nutritional supplement according to an embodiment of the invention. First, at step S1, a protein, slurry is mixed, the protein slurry comprising water and a protein source including milk protein isolate. Generally, the water is heated to between about 120 degrees and about 190 degrees Fahrenheit, preferably to between about 125 degrees and about 175 degrees Fahrenheit, more preferably to between about 130 degrees and about 150 degrees Fahrenheit, and even more preferably to between about 135 degrees and about 145 degrees Fahrenheit, before being combined with the protein source. The protein slurry may further comprise any number of other commonly utilized ingredients, including, for example, antifoaming agents, citrate(s), a fat source, a carbohydrate source, emulsifiers, vitamins, minerals, and flavors. Preferably, the protein slurry of step S1 includes at least heated water and a protein source including milk protein isolate. If an anti-foaming agent is to be used, it is preferably included in the protein slurry of step S1.

At optional step S2, commonly utilized ingredients not included in the protein slurry of step S1, or additional quantities of ingredients so added, are added to the protein slurry and mixed. As noted above, such ingredients may include, for example, citrate(s), a fat source, a carbohydrate source, emulsifiers, vitamins, minerals, and flavors. If one or more citrates are to be added to the combined ingredients, they are preferably added during steps S1 or S2.

At step S3, the combined ingredients of step S1 and, optionally, step S2, are subjected to direct steam injection (DSI). Preferably, the DSI step of the present invention includes heating the combined ingredients to about 250 degrees Fahrenheit for about 45 seconds, followed by flash cooling in a vacuum chamber to about 160 degrees Fahrenheit.

At optional step S4, ingredients not added at steps S1 or S2, or additional quantities of ingredients so added, are added to the combined ingredients. As noted above, such ingredients may include, for example, a fat source, a carbohydrate source, emulsifiers, vitamins, minerals, and flavors. If a fat source, carbohydrate source, or emulsifiers are to be added, they are preferably added during steps S1, S2, or S4.

At step S5, the combined ingredients are homogenized. Any homogenization method may be employed. However, the preferred homogenization method of the present invention is a two-stage homogenization at about 2500 p.s.i. and about 500 p.s.i., respectively. Optionally, step S5 may include more than one homogenization of the combined ingredients.

At optional step S6, ingredients not added at steps S1, S2, or S4, or additional quantities of ingredients so added, are added to the combined ingredients. As noted above, such ingredients may include, for example, vitamins, minerals, and flavors.

At optional step S7, the combined ingredients are again homogenized. The homogenization method and/or parameters of optional step S7 may be the same as or different than those of step S5. If employed, step S7 includes cooling the combined ingredients to less than about 45 degrees Fahrenheit following homogenization. If optional step S7 is not employed, the combined ingredients may be cooled to less than about 45 degrees Fahrenheit following the homogenization of step S5 or after adding additional ingredients at optional step S6. In any case, the combined ingredients may optionally be maintained for up to about 36 hours at less than about 45 degrees Fahrenheit.

Finally, the combined ingredients are subjected to either an aseptic process at step S8 or a retort process at step S9. The aseptic process of step S8 may include, for example, ultra-high temperature (UHT) processing or DSI processing to a temperature greater than about 280 degrees Fahrenheit, followed by sterile homogenization. The finished supplement may then be aseptically filled into briks, cans, bottles, or any other suitable container, as known in the art.

The retort process of step S9 may include, for example, filling the finished supplement into cans, bottles, or any other suitable container, as known in the art, then retorting for sterility.

Supplements prepared according to the method of FIG. 1 have a lower viscosity than known supplements containing milk protein isolate. Generally supplements prepared according to the invention have a viscosity of less than about 120 centipoises, preferably less than about 100 centipoises, more preferably between about 50 centipoises and about 90 centipoises, and most preferably between about 50 centipoises and about 75 centipoises. In addition, treatment of milk protein isolate or other protein sources with DSI early in the manufacturing process greatly reduces the viscosity of the combined ingredients during the remainder of the manufacturing process. In some cases, preprocess viscosity may be reduced by as much as 500 to 1000 centipoises at 50 degrees Fahrenheit. This is a great advantage in large-scale manufacturing of a supplement, as it enables easy movement of the supplement from one stage of the manufacturing process to another, such as, for example, pumping the supplement from a holding tank to a site of final homogenization or sterilization.

The following examples are illustrative, non-limiting methods of supplement preparation according to the invention:
Example 1
   Heat water to 140-180°F. Add protein and antifoam; mix.
   Add citrate[s] and mix.
   DSI at 250°F for 45 sec and flash cool in vacuum chamber to ~160°F.
   Add carbohydrates and fats/emlsifiers; mix.
   Add vitamins, minerals, and flavors; mix.
   Homogenize at 2500/500 psi.
   Cool to < 45°F.
   Product can be stored for O-36 hours at < 45°F.
   UHT or DSI at > 280°F and sterile homogenize.
   Fill aseptically into briks, cans or bottles.
Example 2
   Heat water to 140-180°F. Add protein and antifoam; mix.
   DSI at 250°F for 45 sec and flash cool in vacuum chamber to ~160°F.
   Add carbohydrates and fats/emulsifiers; mix.
   Add vitamins, minerals, and flavors; mix.
   Homogenize at 2500/500 psi.
   Cool to < 45°F.
   Product can be stored for 0-36 hours at < 45°F.
   UHT or DSI at > 280°F and sterile homogenize.
   Fill aseptically into briks, cans or bottles.
Example 3
   Heat water to 140-180°F. Add protein, fat, emulsifiers and antifoam; mix. DSI at 250°F for 45 sec and flash cool in vacuum chamber to ~160°F. Homogenize at 2500/500.
   Add carbohydrates; mix.
   Add vitamins, minerals, and flavors; mix.
   Homogenize at 2500/500 psi.
   Cool to < 45°F.
   Product can be stored for O-36 hours at < 45°F.
   UHT or DSI at > 280°F and sterile homogenize.
   Fill aseptically into briks, cans or bottles.
Example 4
   Heat water to 140-180°F. Add protein, fat, carbohydrate, emulsifiers and antifoam; mix.
   DSI at 250°F for 45 sec and flash cool in vacuum chamber to ~160°F. Homogenize at 2500/500.
   Add vitamins, minerals, and flavors; mix.
   Cool to < 45°F.
   Product can be stored for 0-36 hours at < 45°F.
   UHT or DSl at > 280°F and sterile homogenize.
   Fill aseptically into briks, cans or bottles.
Example 5
   Heat water to 140-180°F. Add protein, carbohydrate, and antifoam: mix.
   DSI at 250°F for 45 sec and flash cool in vacuum chamber to ~160°F. Add fat and emulsifiers.
   Homogenize at 2500/500.
   Add vitamins, minerals, and flavors; mix.
   Cool to < 45°F.
   Product can be stored for O-36 hours at < 45°F.
   UHT or DSI at > 280°F and sterile homogenize.
   Fill aseptically into briks, cans or bottles.
Example 6
   Heat water to 140-180°F. Add protein, carbohydrate, fat, emulsifiers, minerals, some vitamins and antifoam; mix.
   DSI at 250°F for 45 sec and flash cool in vacuum chamber to ~160°F. Homogenize at 2500/500.
   Add vitamins and flavors; mix.
   Cool to < 45°F.
   Product can be stored for 0-36 hours at < 45°F.
   UHT or DSI at > 280°F and sterile homogenize.
   Fill aseptically into briks, cans or bottles.
Example 7
   Heat water to 140-180°F. Add protein and antifoam; mix.
   Add citrate(s) and mix.
   DSI at 250°F for 45 sec and flash cool in vacuum chamber to ~160°F.
   Add carbohydrates and fats/emulsifiers; mix.
   Add vitamins, minerals; and flavors; mix.
   Homogenize at 2500/500 psi twice.
   Cool to < 45°F.
   Product can be stored for 0-36 hours at < 45°F.
   Fill cans or bottles and retort.
Example 8
   Heat water to 140-180°F. Add protein and antifoam; mix.
   DSI at 250°F for 45 sec and flash cool in vacuum chamber to ~160°F. Add carbohydrates and fats/emulsifiers; mix.
   Add vitamins, minerals, and flavors; mix.
   Homogenize at 2500/500 psi twice.
   Cool to < 45°F.
   Product can be stored for O-36 hours at < 45°F.
   Fill cans or bottles and retort.
Example 9
   Heat water to 140-180°F. Add protein, fat, emulsifiers and antifoam; mix. DSI at 250°F for 45 sec and flash cool in vacuum chamber to ~160°F. Homogenize at 2500/500.
   Add carbohydrates; mix.
   Add vitamins, minerals, and flavors; mix.
   Homogenize at 2500/500 psi.
   Cool to < 45°F.
   Product can be stored for O-36 hours at < 45°F.
   Fill cans or bottles and retort.
Example 10
   Heat water to 140-180°F. Add protein, fat, carbohydrate, emulsifiers and antifoam; mix.
   DSI at 250°F for 45 sec and flash cool in vacuum chamber to ~160°F. Homogenize at 2500/500.
   Add carbohydrates; mix.
   Add vitamins, minerals, and flavors; mix.
   Homogenize at 2500/500.
   Cool to < 45°F.
   Product can be stored for O-36 hours at < 45°F.
   Fill cans or bottles and retort.
Example 11
   Heat water to 140-180°F. Add protein, carbohydrate, and antifoam; mix. DSI at 250°F for 45 sec and flash cool in vacuum chamber to ~160°F. Add fat and emulsifiers.
   Homogenize at 2500/500 twice.
   Add vitamins, minerals, and flavors; mix.
   Cool to < 45°F.
   Product can be stored for O-36 hours at < 45°F.
   Fill cans or bottles and retort.
Example 12
   Heat water to 140-180°F. Add protein, carbohydrate, fat, emulsifiers, minerals, some vitamins and antifoam; mix.
   DSI at 250°F for 45 sec and flash cool in vacuum chamber to ~160°F.
   Homogenize at 2500/500 twice
   Add vitamins and flavors; mix.
   Cool to < 45°F.
   Product can be stored for O-36 hours at < 45°F.
   FII cans or bottles and retort.

### High-Calorie Nutritional Supplement

The invention further provides nutritional supplements comprising a protein source including milk protein isolate and/or canola plant protein and having a viscosity suitable for oral administration. Nutritional supplements according to the invention may be produced according to a method above or any other method.

Nutritional supplements according to the invention are generally calorically dense, providing between about 2.25 and about 3.25 calories per milliliter. A protein source provides between about 10% and about 22% of the'total calories of the supplement, a fat source provides between about 34% and about 55% of the total calories of the supplement, and a carbohydrate source provides between about 25% and about 55% of the total calories of the supplement.

Table 1 shows typical ingredient ranges of nutritional supplements according to the invention.

**Table 1 - Illustrative Ingredient Ranges**

| | 2.25-3.25 calorie per mL | % US RDI per serving |
|---|---|---|
| Serving Size | 237 ml | - |
| Kcalories | 530-770 | - |
| Caloric Density (cal/ml) | 2.25-3.25 | - |
| Total Protein g | 16-30 | - |
| Fat g | 23-36 | - |
| Carbohydrate g | 38-90 | - |
| Protein % calories | 10-22 | - |
| Fat % calories | 34-55 | - |
| Carbohydrate % calories | 25-55 | - |
| Fiber (g) | 0-3.0 | - |
| Vit A (IU) | 0-2500 | 0-50 |
| Vit C (mg) | 0-90 | 0-150 |
| Vit D (IU) | 0-200 | 0-50 |
| Vit E (IU) | 0-30 | 0-100 |
| Vit K (mcg) | 0-40 | 0-50 |
| B6 (mg) | 1.00 | 0-50 |
| B12 (mcg) | 0-1.0 | 0-50 |
| Thiamine (mg) | 0-0.76 | 0-50 |
| Riboflavin (mg) | 0-1.02 | 0-50 |
| Folic Acid (mcg) | 0-200 | 0-50 |
| Pantothenic Acid (mg) | 0-5.0 | 0-50 |
| Choline (mg) | 0-284 | - |
| Biotin (mcg) | 0-150 | 0-50 |
| Niacin (mg) | 0-10.0 | 0-50 |
| Calcium (mg) | 0-500 | 0-50 |
| Iron (mg) | 0-10.8 | 0-50 |
| Manganese (mg) | 0-1.0 | 0-50 |
| Chloride (mg) | 0-700 | 0-20 |
| Phosphorus (mg) | 0-500 | 0-50 |
| Iodine (mcg) | 0-75 | 0-50 |
| Magnesium (mg) | 0-200 | 0-50 |
| Zinc (mg) | 0-7.5 | 0-50 |
| Copper (mg) | 0-1.0 | 0-50 |
| Sodium (mg) | 0-1200 | 0-50 |
| Potassium (mg) | 0-875 | 0-25 |
| Chromium (mcg) | 0-60 | 0-50 |
| Molybdenum (mcg) | 0-37.6 | 0-50 |
| Selenium (mg) . | 0-50 | 0-50 |

Nutritional supplements according to the invention include milk protein isolate and/or canola plant protein as a primary or sole protein source. As noted above, when prepared according to a method of the invention, DSI reduces the viscosity of the supplement such that milk protein isolate may be used as a protein source, thus avoiding the deficiencies of known supplements. Nutritional supplements according to the invention may also include one or more of the following as a protein source: total milk proteins, milk protein concentrate, soy protein isolate, whey hydrolysate, and free amino acids. The protein source may include Canola plant proteins including Supertein, the 2S fraction and Puratein, the 12S fraction from canola seed proteins from ADM. The protein source may include branched chain amino acids (BCAAs) (i.e., leucine, isoleucine, and/or valine) in any number of forms, including, for example, as free BCAAs; in intact proteins; as dipeptides, tripeptides, fractionated protein, or hydrolyzed protein; in enriched form (protein isolate); and/or in salt.form. BCAAs may similarly be included in any combination of the above. If included in a nutritional supplement of the invention, BCAAs preferably make up at least about 5% to about 50% of the weight of the amino-nitrogen source (i.e., % of total amino acids and therefore % of total calories of the protein source), more preferably at least about 10% to about 40% of such weight, even more preferably at least about 15% to about 35% of such weight, and most preferably at least about 22% to about 35% of such weight.

In addition, nutritional supplements according to the invention may further comprise a fat source (e.g., one or more of canola oil, corn oil, cottonseed oil, soybean oil, soy lecithin, high-oleic sunflower oil, sunflower oil, fish oil, medium chain triglycerides (MCTs), high-oleic safflower oil, safflower oil, olive oil, borage oil, black currant oil, evening primrose oil, flaxseed oil, palm kernel oil, and coconut oil). Preferably, fish oil may be used, such as concentrated fish oil comprising about 70% eicosapentawnoic acid [EPA], or an oil comprising about 45% EPA and about 10% docosahexaenoic acid (DHA). The latter oil includes those commercially available, for example, under the tradename EPAX^{®} 4510 from Pronova biocare. In one embodiment, a composition according to the invention includes between about 2.5 g and about 7.5 g of fish oil per serving, more preferably between about 3.5 g and about 6.5 g, even more preferably between about 4.5 g and about 5.5 g, and most preferably about 5.5 g. Such a composition may alternatively or also include between about 0.5 g and about 5 g of MCT per serving, preferably between about 1 g and about 4.5 g, more preferably between about 1.5 g and about 4 g, even more preferably between about 2 g and about 3.5 g, even more preferably between about 2.5 g and about 3 g. Typically, two to three servings of compositions according to the invention may be administered per day, although as many as five to six servings may be administered.

Many of the fat sources above include n-3 polyunsaturated (Omega-3) fatty acids, such as alpha-linolenic acid (LNA), EPA, and DHA, either alone or in combination. In one embodiment, a composition according to the invention, includes fish oil providing between about 0.5 g and about 3 g of n-3 polyunsaturated fatty acids, preferably between about 1.5 g and about 2 g of n-3 polyunsaturated fatty acids.

EPA may be present alone, for example, in an amount of at least about 600 mg to about 2 g per serving, preferably at least about 1.5 g to about 1.8 g per serving. EPA and DHA may be present in combination, for example, wherein EPA is present in an amount between about 500 mg and about 1.5 g, preferably about 1.0 g per serving, while DHA is present in an amount between about 250 mg and about 1.5 g, preferably between about 500 mg and about 750 mg, and more preferably about 650 mg per serving. In one embodiment, a composition according to the invention includes both EPA and DHA in a ration of about 2:1, about 1:2, or about 1.5:1.

In another embodiment, a composition of the invention may include a mixture of n-6 polyunsaturated fatty acids, such as linoleic acid, and one or more n-3 polyunsaturated fatty acid, such as LNA, EPA, and DHA. Such n-6 and n-3 polyunsaturated acids may be present, for example in a ratio between about 0.1:1.0 and about 1.0:0.1 (e.g., 0.2:1.0, 0.5:1.0, 0.8:1.0, 1:1, 1.2:1.0, and 1.5:1.0, more preferably about 1.1:1.0).

Optionally, a composition according to the invention may includes monounsaturated fatty acids (MUFA). When so included, a composition according to the invention preferably includes between about 2 g and about 3.5 g of MUFA per serving, more preferably between about 2.5 g and about 3 g of MUFA per serving and between about 3 g and about 6 g of polyunsaturated fatty acids per serving, more preferably between about 4.5 g and about 5 g of polyunsaturated fatty acids per serving.

Nutritional supplements according to the invention may also further comprise a carbohydrate source (e.g., one or more of sugar, liquid sucrose, maltodextrin, corn syrup solids, high fructose corn syrup, corn syrup, a soluble fiber, trehalose, isomaltulose, and fructose). When provided as a nutritionally- dense formula, a composition according to the invention preferably includes a slowly digested or slowly metabolizable sugar, such that metabolism is prolonged and results in a lower insulinogenic response. The use of such sugars may improve insulin sensitivity, reduce blood/plasma glucose concentrations, and improve metabolic response, including improved nitrogen balance and endogenous protein synthesis. Suitable sugars include, for example, isomalt, isomaltulose, trehelose, D-tagatose, tapioca dextrin, and sucromalt.

Nutritional supplements according to the invention may also further comprise vitamins (e.g., one or more of A, C, D, E, K, B6, B12, thiamine, riboflavin, folic acid, pantothenic acid, biotin, and choline] and minerals (e.g., one or more of niacin, calcium, iron, manganese, chloride, phosphorus, iodine, magnesium, zinc, copper, sodium, potassium, chromium, molybdenum, and selenium).

Peptides derived from milk protein have been shown to possess various bioactive properties, including anti-hypertensive and anti-thrombotic properties. Such peptides may also serve as mineral carriers. Both casein- and whey-derived peptides may provide additional physiological and/or medical benefits. For example, some peptides released from casein during digestion can influence gastrointestinal motility, prolong gastrointestinal transit time, and exert antidiarrheal actions. This can be particularly beneficial to individuals suffering from weight loss caused by an illness or the treatment of an illness. Casein phosphopeptides may also prevent the precipitation of calcium phosphate, thereby increasing the concentration of soluble calcium in the lumen of the small intestine. In addition, bioactive compounds and amino acids in whey protein may improve immune function and gastrointestinal health and may also function as antioxidants.

As a further aid to gastrointestinal health, compositions according to the invention may further comprise soluble and/or insoluble fiber. Increased fiber intake has been shown to provide numerous benefits, including reduced transit time; increased stool weight; improved stool evacuation; improved barrier protection to the gut (thereby preventing bacteria entering the bloodstream); improved fermentation and production of short-chain fatty acids (SCFAs), an energy source for intestinal mucosal cells; and stimulation of immune cells of the gastrointestinal tract.

The addition of fiber to compositions of the invention may be particularly beneficial for individuals requiring additional calories and protein and who may be treated with a "medication pass program," described in greater detail below.

Suitable fibers for inclusion in compositions of the invention include insoluble fibers and soluble fibers. Insoluble fibers, including cellulose, some hemicelluoses, and lignin, are completely insoluble in water and are minimally fermented in the colon. They exert their beneficial effects primarily as bulking agents, through their capacity to hold water. Insoluble fibers increase stool mass and shorten transit time of stool passing through the intestinal tract, aiding in the prevention or alleviation of constipation. In addition, they-may increase excretion of bile acids. Cellulose and hemicellulose are found in the stalks and leaves of vegetables and outer covering of seeds, while lignin is found in the stems and seeds of fruits and vegetables, and in the bran layer of cereals. Significant amounts of insoluble fiber are also found in soy, wheat bran and other whole grain cereals.

Soluble fibers are soluble in water and are fermented in the colon. The amount of fermentation depends on the degree of solubility and the particle size of the fibers. Thus, as the solubility of a fiber increases and its particle size decreases, it is more rapidly and completely fermented. Beneficial effects of soluble fibers include delaying gastric emptying, delaying the absorption of some nutrients in the small intestine, and lowering serum cholesterol levels. Soluble fibers include pectin, gums, some hemicelluloses, psyllium, guar gum, fructooligosaccharides [FOS], inulin, and galactooligosaccharides (GOS). In addition, significant amounts of soluble fiber are found in fruits, vegetables, and cereal such as barley and oats.

During the fermentation process, SCFAs are produced and are thought to be responsible for some of the beneficial effects attributed to fiber. Acetate, propionate, and butyrate comprise 83% of the SCFAs produced in the colon. SCFAs are readily absorbed by the intestinal mucosa and metabolized in the intestine and liver; providing energy; stimulating sodium and water absorption in the colon, and promoting intestinal growth. Due to these properties, SCFAs are thought to reduce the risk and frequency of diarrhea induced by illness, drugs, and bacterial contamination of the gut. Butyrate is the preferred fuel for mucosal cells in the colon and is important for maintaining the intestinal mucosa, which is a major barrier to the invasion of bacteria into the bloodstream. SCFAs also promote a healthy gut environment. They inhibit the growth of harmful bacteria and stimulate-the growth of beneficial bacteria, such as bifidobacteria and lactobacilli. Beneficial bacteria have a number of important functions in the gastrointestinal tract, including promotion of intestinal health, stimulation of immune responses, and inhibition of the growth of harmful bacteria, especially during antibiotic therapy. Through the stimulation of bacterial growth and fermentation, soluble fibers have a mild laxative effect and can be helpful in preventing or alleviating constipation.

Some examples of other fibers to optionally use in accordance with this invention are those derived from bran and seeds; cellulose; chitin; chitosan; hemicellulose; lignin; carbohydrates [for example: beta-galactooligosaccharides, alpha-galactooligosaccharides, fructo-oligosaccharide, fuco-oligosaccharides, manno-otigosaccharides, xylo-oligosaccharides, sialyl-oligosaccharides, N-glycoprotein oligosaccharides, O-glycoprotein oligosaccharides, glycolipid oligosaccharides, cello- oligosaccharides, chitosan- oligosaccharides, chitin-oligosaccharides; galactourono- oligosaccharides, glucourono- oligosaccharides, beta-glucan oligosaccharides, arabinoxylo- oligosaccharides, arabinogalactooligosaccharides, xylogluco- oligosaccharides, galactomanno- oligosaccharides, rhamno- oligosaccharides]; soluble carbohydrates or saccharides [for example: fructo(os)anes/inulins, galatans, fucoidans, arabinas, zylans, xanthans, beta-glucans, galacturonans; N-glycans, O-glycans_{;} hyaluronic acids, chondroitins, xyloglucans, arabinogalactans, alginates, carageenanes, galactomannans, arabinoxylanes, glycolipid glycans, glycoprotein glycans, proteoglycans, reserve carbohydrates, fructans, galacto oligosaccharides from legumes, fucoidan, alpha-glucane, laminarin, carragenan, mannans, galactomannans, agar, natural gum, N-glycosidic bonded carbohydrates of glycoproteins, glycans of glycolipids, enzymatically prepared carbohydrates (galacto- / gluco- / xylo - oligosaccharides), bacterial carbohydrates (xanthan), oligosaccharides: galacto-/ gluco- (from alpha 1-2 and alpha 1-3 glucose residues), xylo-oligosaccharides, celluloses, hemicelluloses [arabians, galactans], pectins, chitins; D-glucose: D-fructose; D-galactose; D-mannose; L-fucose: D-N-acetyl glucosamine; D-N-acetyl galactosamine; D-xylose: L-rhamnose; D-arabinose; D-allose; D-talose; L-idose; D-ribose; D-galacturon acid; D-glucuron; and D-mannuron acid.

Tables 2-4 below, provide illustrative examples of nutritional supplement formulations according to the invention. The formulations shown have high caloric values of 2.25, 2.5, and 3.0 calories per mL, although higher and lower caloric values are also possible and within the scope of the invention. Preferably, nutritional supplements according to the invention have a caloric value of between about 2.25 and about 3.25 calories per mL.

**Table 2 - Illustrative 2.25 Calorie / mL Formulations**

| | 2.25 calorie per mL | % US RDI | 2.25 calorie per mL | % US RDI | 2.25 calorie per mL | % US RDI | 2.25 calorie per mL | % US RDI |
|---|---|---|---|---|---|---|---|---|
| Serving Size (mL) | 237 | - | 237 | - | 237 | - | 237 | - |
| Kcalories (cal) | 535 | - | 535, | - | 535 | - | 535 | - |
| Caloric Density (cal/mL) | 2.25 | - | 2.25 | - | 2.25 | - | 2.25 | - |
| Total Protein g | 24.0 | - | 20.0 | - | 16.0 | - | 30.0 | - |
| Fat g | 23.0 | - | 26.0 | - | 29.0 | - | 24.0 | - |
| Carbohydrate g | 58.0 | - | 55.0 | - | 52.5 | - | 49.5 | - |
| Protein % calories | 18 | - | 15 | - | 12 | - | 15 | - |
| Fat % calories | 39 | - | 44 | - | 49 | - | 44 | - |
| Carb % calories | 43 | - | - | - | 39 | - | 41 | - |
| Fiber (g) | 0 | - | 0 | - | 0 | - | 0 | 0 |
| Vit A (IU) | 1250 | 25.0 | 1250 | 25.0 | 1250 | 25.0 | 1250 | 25.0 |
| Vit C (mg) | 90 | 150.0 | 90 | 150.0 | 90 | 150.0 | 90 | 150.0 |
| Vit D (IU) | 100 | 25.0 | 100 | 25.0 | 100 | 25.0 | 100 | 25.0 |
| Vit E (IU) | 30 | 100.0 | 30 | 100.0 | 30 | 100.0 | 30 | 100:0 |
| Vit K (mcg) | 20 | 25.0 | 20 | 25.0 | 20 | 25.0 | 20 | 25.0 |
| B6 (mg) | 0.60 | 30.0 | 0.60 | 30.0 | 0.60 | 30.0 | 0.60 | 30.0 |
| B12 (mcg) | 1.5 | 25.0 | 1.5 | 25.0 | 1.5 | 25.0 | 1.5 | 25.0 |
| Thiamine (mg) | 0.38 | 25.3 | 0.38 | 25.3 | 0.38 | 25.3 | 25.3 | 25.3 |
| Riboflavin (mg) | 0.51 | 30.0 | 0.51 | 30.0 | 0.51 | 30.0 | 0.51 | 30.0 |
| Folic Acid (mcg) | 100 | 25.0 | 100 | 25.0 | 100 | 25.0 | 100 | 25.0 |
| Pantothenic Acid (mg) | 2.5 | 25.0 | 2.5 | 25.0 | 2.5 | 25.0 | 2.5 | 25.0 |
| Choline (mg) | 142 | - | 142 | - | 142 | - | 142 | - |
| Biotin (mcg) | 75 | 25.0 | 75 | 25.0 | 75 | 25.0 | 75 | 25.0 |
| Niacin (mg) | 5.0 | 25.0 | 5.0 | 25.0 | 5.0 | 25.0 | 5.0 | 25.0 |
| Calcium (mg) | 450 | 45.0 | 450 | 45.0 | 450 | 45.0 | 450 | 45.0 |
| Iron (mg) | 5.4 | 30.0 | 5.4 | 30.0 | 5.4 | 30.0 | 5.4 | 30.0 |
| Manganese (mg) | 0.5 | 25.0 | 0.5 | 25.0 | 0.5 | 25.0 | 0.5 | 25.0 |
| Chloride (mg) | 350 | 10.3 | 350 | 10.3 | 350 | 10.3 | 350 | 10.3 |
| Phosphorus (mg) | 350 | 35.0 | 350 | 35.0 | 350 | 35.0 | 350 | 35.0 |
| Iodine (mcg) | 37.5 | 25.0 | 37.5 | 25.0 | 37.5 | 25.0 | 37.5 | 25.0 |
| Magnesium (mg) | 100 | 25.0 | 100 | 25.0 | 100 | 25.0 | 100 | 25.0 |
| Zinc (mg) | 4.5 | 30.0 | 4.5 | 30.0 | 4.5 | 30.0 | 4.5 | 30.0 |
| Copper (mg) | 0.7 | 35.0 | 0.7 | 35.0 | 0.7 | 35.0 | 0.7 | 35.0 |
| Sodium (mg) | 190 | 7.9 | 190 | 7.9 | 190 | 7.9 | 190 | 7.9 |
| Potassium (mg) | 360 | 10.3 | 360 | 10.3 | 360 | 10.3 | 360 | 10.3 |
| Chromium (mcg) | 30 | 25.0 | 30 | 25.0 | 30 | 25.0 | 30 | 25.0 |
| Molybdenum (mcg) | 18.8 | 25.1 | 18.8 | 25.1 | 18.8 | 25.1 | 18.8 | 25.1 |
| Selenium (mcg) | 17.5 | 25.0 | 17.5 | 25.0 | 17.5 | 25.0 | 17.5 | 25.0 |

**Table 3 - Illustrative 2.5 Calorie / mL Formulations**

| | 2.5 calorie per mL | %US RDI | 2.5 calorie per mL | % US RDI | 2.5 calorie per mL | % US RDI | 2.5 calorie per mL | %US RDI |
|---|---|---|---|---|---|---|---|---|
| Serving Size (mL) | 37 | - | 237 | - | 237 | - | 237 | - |
| Kcalories (cal) | 595 | - | 595 | - | 595 | - | 595 | - |
| Caloric Density (cal/ml) | 2.5 | - | 2.5 | - | 2.5 | - | 2.5 | - |
| Total Protein g | 26.0 | - | 22.0 | | 20.0 | - | 22.5- | - |
| Fat g | 24.0 | - | 26.5 | - | 28.0 | - | 28.0 | - |
| Carbohydrate g | 68.5 | - | 67.0 | - | 65.5 | - | 65.5 | - |
| Protein % calories | 17 | - | 15 | - | 13 | - | 15 | - |
| Fat % calories | 36 | - | 40 | - | 42 | - | 42 | - |
| Carb % calories | 46 | - | 45 | - | 44 | - | 42 | - |
| Fiber (g) | 0 | - | 0 | - | 0 | - | 0 | - |
| Vit A (IU) | 1250 | 25.0 | 1250 | 25.0 | 1250 | 25.0 | 1250 | 25.0 |
| Vit C (mg) | 90 | 150.0 | 90 | 150.0 | 90 | 150.0 | 90 | 150.0 |
| Vit D (IU) | 100 | 25.0 | 100 | 25.0 | 100 | 25.0 | 100 | 25.0 |
| Vit E (IU) | 30 | 100.0 | - | 100.0 | 30 | 100.0 | 30 | 100.0 |
| Vit K (mcg) | 20 | 25.0 | 20 | 25.0 | 20 | 25.0 | 20 | 25.0 |
| B6 (mg) | 0.60 | 30.0 | 0.60 | 30.0 | 0.60 | 30.0 | 0.60 | 30.0 |
| B12 (mcg) | 1.5 | 25.0 | 1.5 | 25.0 | 1.5 | 25.0 | 1.5 | 25.0 |
| Thiamine (mg) | 0.38 | 25.3 | 0.38 | 25.3 | 0.38 | 25.3 | 0.38 | 25.3 |
| Riboflavin (mg) | 0.51 | 30.0 | 0.51 | 30.0 | 0.51 | 30.0 | 0.51 | 30.0 |
| FolicAcid (mcg) | 100 | 25.0 | 100 | 25.0 | 100 | 25.0 | 100 | 25.0 |
| Pantothenic Acid (mg) | 2.5 | 25.0 | 2.5 | 25.0 | 2.5 | 25.0 | 2.5 | 25.0 |
| Choline (mg) | 142 | - | 142 | - | 142 | - | 142 | - |
| Biotin (mcg) | 75 | 25.0 | 75 | 25.0 | 75 | 25.0 | 75 | 25.0 |
| Niacin (mg) | 5.0 | 25.0 | 5.0 | 25.0 | 5.0 | 25.0 | 5.0 | 25.0 |
| Calcium (mg) | 450 | 45.0 | 450 | 45.0 | 450 | 45.0 | 450 | 45.0 |
| Iron (mg) | 5.4 | 30.0 | 5.4 | 30.0 | 5.4 | 30.0 | - | 30.0 |
| Manganese (mg) | 0.5 | 25.0 | 0.5 | 25.0 | 0.5 | 25.0 | 0.5 | 25.0 |
| Chloride (mg) | 350 | 10.3 | 350 | 10.3 | 350 | 10.3 | 350 | 10.3 |
| Phosphorus (mg) | 350 | 35.0 | 350 | 35.0 | 350 | 35.0 | 350 | 35.0 |
| Iodine (mcg) | 37.5 | 25.0 | 37.5 | 25.0 | 37.5 | 25.0 | 37.5 | 25.0 |
| Magnesium (mg) | 100 | 25.0 | 100 | 25.0 | 100 | 25.0 | 100 | 25.0 |
| Zinc (mg) | 4.5 | 30.0 | 4.5 | 30.0 | 4.5 | 30.0 | 4.5 | 30.0 |
| Copper (mg) | 0.7 | 35.0 | 0.7 | 35.0 | 0.7 | 35.0 | 0.7 | 35.0 |
| Sodium (mg) | 190 | 7.9 | 190 | 7.9 | 190 | 7.9 | 190 | 7.9 |
| Potassium (mg) | 360 | 10.3 | 360 | 10.3 | 360 | 10.3 | 360 | 10.3 |
| Chromium (mcg) | 30 | 25.0 | 30 | 25.0 | 30 | 25.0 | 30 | 25.0 |
| Molybdenum (mcg) | 18.8 | 25.1 | 18.8 | 25.1 | 18.8 | 25.1 | 18.8 | 25.1 |
| Selenium (mcg) | 17.5 | 25.0 | 17.5 | 25.0 | 17.5 | 25.0 | 17.5 | 25.0 |

**Table 4 - Illustrative 2.5 Calorie / mL and 3.0 Calorie / mL Formulations**

| | 2.50 calorie per mL | % US RDI | 3.0 calorie per mL | % US RDI | 3.0 calorie per mL | % US RDI | calorie per mL | % US RDI |
|---|---|---|---|---|---|---|---|---|
| Serving Size (mL) | 237 | - | 237 | | 237 | - | 237 | - |
| Kcalories (cal) | 595 | - | 710 | - | 710 | - | 710 | - |
| Caloric Density (cal/ml) | 2.5 | - | 3.0 | - | 3.0 | - | 3.0 | - |
| Total Protein g | 30.0 | - | 26.0 | - | 24.0 | - | 20.0 | - |
| Fat g | 26.0 | - | 36.0 | - | 32.0 | - | - 30.0 | - |
| Carbohydrate g | 60.0 | - | 71.0 | - | 82.0 | - | 90.0 | - |
| Protein % calories | 20 | - | 15 | - | 14 | - | 11 | - |
| Fat % calories | 39 | - | 46 | 46 - | 41 | - | 38 | - |
| Carb % calories | 40 | - | 40 | - | 46 | - | 51 | - |
| Fiber (g) | 0 | - | 0 | - | 0 | - | 0 | - |
| Vit A (IU) | 1250 | 25.0 | 1250 | 25.0 | 1250 | 25.0 | 1250 | 25.0 |
| Vit C (mg) | 90 | 150.0 | 90 | 150.0 | 90 | 150.0 | 90 | 150.0 |
| Vit D (IU) | 100 | 25.0 | 100 | 25.0 | 100 | 25.0 | 100 | 25.0 |
| Vit E (IU) | 30 | 100.0 | 30 | 100.0 | 30 | 100.0 | 30 | - |
| Vit K (mcg) | 20 | 25.0 | 20 | 25.0 | 20 | 25.0 | 20 | 25.0 |
| B6 (mg) | 0.60 | 30.0 | 0.60 | 30.0 | 0.60 | 30.0 | - | 30.0 |
| B12 (mcg) | 1.5 | 25.0 | 1.5 | 25.0 | 1.5 | 25.0 | 1.5 | 25.0 |
| Thiamine (mg) | 0.38 | 25.3 | 0.38 | 25.3 | 0.38 | 25.3 | 0.38 | 25.3 |
| Riboflavin (mg) | 0.51 | 30.0 | 0.51 | 30.0 | 0.51 | 30.0 | 0.51 | 30.0 |
| Folic Acid (mcg) | 100 | 25.0 | 100 | 25.0 | 100 | 25.0 | 100 | 25.0 |
| Pantothenic Acid (mg) | 2.5 | 25.0 | 2.5 | 25.0 | 2.5 | 25.0 | 2.5 | 25.0 |
| Choline (mg) | 142 | - | 142 | - | 142 | - | 142 | - |
| Biotin (mcg) | 75 | 25.0 | 75 | 25.0 | 75 | 25.0 | 75 | 25.0 |
| Niacin (mg) | 5.0 | 25.0 | 5.0 | 25.0 | 5.0 | 25.0 | 5.0 | 25.0 |
| Calcium (mg) | 450 | 45.0 | 450 | 45.0 | 450 | 45.0 | 450 | 45.0 |
| Iron (mg) | 5.4 | 30.0 | 5.4 | 30.0 | 5.4 | 30.0 | 5.4 | 30.0 |
| Manganese (mg) | 0.5 | 25.0 | - | 25.0 | 0.5 | 25.0 | 0.5 | 250 |
| Chloride (mg) | 350 | 10.3 | 350 | 10.3 | 350 | 10.3 | 350 | - |
| Phosphorus (mg) | 350 | 35.0 | 350 | 35.0 | 350 | 35.0 | 350 | 35.0 |
| Iodine (mcg) | 37.5 | 25.0 | 37.5 | 25.0 | 37.5 | 25.0 | 37.5 | 25.0 |
| Magnesium (mg) | 100 | 25.0 | 100 | 25.0 | 100 | 25.0 | 100 | 25.0 |
| Zinc (mg) | 4.5 | 30.0 | 4.5 | 30.0 | 4.5 | 30.0 | 4.5 | 30.0 |
| Copper (mg) | 0.7 | 35.0 | 0.7 | 35.0 | 0.7 | 35.0 | 0.7 | 35.0 |
| Sodium (mg) | 190 | 7.9 | 190 | 7.9 | 190 | 7.9 | 190 | 7.9 |
| Potassium (mg) | 360 | 10.3 | 360 | 10.3 | 360 | 10.3 | 360 | 10.3 |
| Chromium (mcg) | 30 | 25.0 | 30 | 25.0 | 30 | 25.0 | 30 | 25.0 |
| Molybdenum (mcg) | 18.8 | 25.1 | 18.8 | 25.1 | 18.8 | 25.1 | 18.8 | 25.1 |
| Selenium (mcg) | 17.5 | 25.0 | 17.5 | 25.0 | 17.5 | 25.0 | 17.5 | 25.0 |

As described above, nutritional supplements according to the invention generally have a viscosity of less than 120 centipoises, preferably less than 100 centipoises, more preferably between about 50 centipoises and about 90 centipoises, and most preferably between about 50 centipoises and about 75 centipoises. As such, nutritional supplements according to the invention are suitable for oral or enteral administration. Where the nutritional supplement is an orally-administrable nutritional supplement, it may be desirable to further include one or more flavoring agents.

### Treatment of a Nutritional Deficiency with a High-Calorie Nutritional Supplement

The present invention further includes methods for treating a nutritional deficiency in an individual, comprising administering to the individual a nutritional supplement including: a protein source including milk protein isolate and/or canola plant protein; a fat source; and a carbohydrate source, wherein the nutritional supplement has a caloric content of between about 2.25 and about 3.25 calories per milliliter. The nutritional deficiency may be associated with a disease, disorder, or medical condition. Administration of the nutritional supplement may be via oral or enteral administration.

Diseases, disorders, and medical conditions suitable for treatment according to the invention include, for example, anorexia nervosa; wasting diseases, including cancer, acquired immune deficiency syndrome (AIDS), and sarcopenia (age-related loss.of muscle mass); chronic illness; functional limitations, including psychological disorders and physical incapacitation; diminished cognitive ability; and wounds. High-calorie, high-protein nutritional supplements, such as those provided by.an embodiment of the present invention, are particularly beneficial in treating muscle loss associated with the above and other conditions.

As noted above, compositions according to the invention may include protein sources composed, at least partially, of branched chain amino acids (BCAAs). BCAAs, as well as insulin and insulin-like growth factor 1 (IGF-1) have been shown to inhibit the degradation of protein caused by Proteolysis Inducing Factor (PIF). As a result, evidence supports the therapeutic use of BCAAs alone or in combination with either or both of insulin and IGF-1. In addition, PIF-induced protein degradation has reportedly been reduced by eicosapentanoic acid (EPA). BCAAs may also inhibit proteins degradation caused by factors ofther than PIF, further supporting their therapeutic use in the treatment of, among other things, protein degradation. Surprisingly, it was found that each BCAA (leucine, isoleucine, and valine) is equally effective in antagonizing protein degradation. These results are particularly interesting in view of previous research, which focused on the ability of specific amino acids (e.g., leucine) to initiate and promote the synthesis of proteins through upregulation of the synthetic machinery. The goal of such research was the improvement of skeletal muscle anabolism.

Methods of treatment according to the present invention may further include the administration of a medicament-or other therapeutic agent. In one embodiment, a nutritional supplement according to the invention is included in a "medication pass program," wherein a small quantity of a nutritional supplement is administered to an individual in conjunction with one or more medicaments. In a preferred embodiment, such co-administration is periodic, providing regular, small quantities of the nutritional supplement. Such a treatment method is particularly beneficial to individuals sensitive to large volumes of food or nutritional supplements, and who are therefore at an increased risk of malnutrition or weight loss. Thus, administration of a nutritional supplement according to the invention as part of a "medication pass program" can encourage compliance with administration of both the nutritional supplement and the medication, provide calories and protein for weight maintenance and/or weight gain, provide protein to help support wound healing, avoid nutrient deficiencies associated with inadequate nutritional intake, lower the risk of malnutrition and its associated complications, improve appetite, avoid early satiety, and help improve an individual's overall dietary intake. In addition, such administration may reduce waste associated with non-compliance and reduce costs associated with malnutrition and weight loss.

The foregoing description of various aspects of the invention has been presented for purposes of illustration and description. It is not intended to be exhaustive or to limit the invention to the precise form disclosed, and obviously, many modifications and variations are possible. Such modifications and variations that may be apparent to a person skilled in the art are intended to be included within the scope of the invention as defined by the accompanying claims.

## Claims

1. A nutritional supplement comprising:
a protein source comprising milk protein isolate or canola plant protein or milk protein isolate and canola plant protein;
a fat source; and
a carbohydrate source,
wherein the nutritional supplement has a caloric content of between about 2.25 and about 3.25 calories per milliliter and has a viscosity of less than about 120 centipoises;
with the proviso that the protein source does not include caseinate.

2. The nutritional supplement of claim 1, wherein the fat source includes at least one of the following: canola oil, corn oil, cottonseed oil, soybean oil, soy lecithin, high-oleic sunflower oil, sunflower oil, fish oil, medium chain triglycerides, high-oleic safflower oil, safflower oil, palm kernel oil, olive oil, borage oil, blackcurrant oil, evening primrose oil, flaxseed oil, and coconut oil.

3. The nutritional supplement of claim 1, wherein the carbohydrate source includes at least one of the following: sugar, liquid sucrose, maltodextrin, corn syrup solids, high fructose corn syrup, corn syrup, a soluble fiber, trehalose, isomaltulose and fructose.

4. The nutritional supplement of claim 1, wherein the protein source is milk protein isolate.

5. The nutritional supplement of claim 1, wherein the protein source further includes at least one of the following: total milk proteins, milk protein concentrate, soy protein isolate, whey hydrolysate, and a free amino acid.

6. The nutritional supplement of claim 1, wherein the protein source is canola plant protein; optionally wherein the canola plant protein includes at least one of the following the 2S fraction from canola seed proteins, the 12S fraction from canola seed proteins or combinations thereof.

7. The nutritional supplement of claim 1, wherein the protein source includes (A) at least one of the following: intact proteins; free peptides; fractionated proteins; and combinations thereof; or (B) at least one branch chain amino acid [BCAA]; optionally wherein the at least one BCAA
(a) includes at least one of an intact BCAA, a partially hydrolyzed BCAA, a salt thereof, and combinations thereof; or
(b) makes up preferably about 5-50% of the calories of the protein; optionally wherein the at least one BCAA makes up (i) preferably about 10-40% of the calories of the protein; or (ii) preferably about 15-35% of the calories of the protein; or
(c) makes up preferably about 22-35% of the calories of the protein.

8. The nutritional supplement of claim 1, wherein:
(A) the protein source provides between about 10% and about 22% of the caloric content of the supplement; optionally wherein
(a) the fat source provides between about 34% and about 55% of the caloric content of the supplement; or
(b) the carbohydrate source provides between about 25% and about 55% of the caloric content of the supplement; or
(B) the fat source includes at least one n-3 polyunsaturated fatty acid selected from a group consisting of: alpha-linolenic acid [LNA], eicosapentaenoic acid [EPA], and docosahexaenoic acid [DHA]; optionally wherein
(a) the fat source includes EPA and DHA in a ratio of about 2: 1; or
(b) the fat source includes EPA and DHA in a ratio of about 1:2; or
(c) the fat source includes EPA and DHA in a ratio of about 1.5:1; or
(d) the fat source further includes at least one n-6 polyunsaturated fatty acid; optionally wherein the fat source includes n-6 polyunsaturated fatty acids and n-3 polyunsaturated fatty acids in a ratio between about 0.1:1.0 and about 1.0:0.1; or
(e) the fat source further includes at least one monounsaturated fatty acid.

9. The nutritional supplement of claim 1, further comprising:
(A) at least one vitamin selected from a group consisting of: A, C, D, E, K, B6, B12, thiamine, riboflavin, folic acid, pantothenic acid, biotin, and choline; or
(B) at least one mineral selected from a group consisting of: niacin, calcium, iron, manganese, chloride, phosphorus, iodine, magnesium, zinc, copper, sodium, potassium, chromium, molybdenum, and selenium; or
(C) a fiber source including at least one of an insoluble fiber and a soluble fiber; optionally wherein the at least one of an insoluble fiber and a soluble fiber is selected from a group consisting of: cellulose; chitin; chitosan; hemicellulose; lignin; beta-galactooligosaccharides, alpha-galactooligosaccharides, fructo-oligosaccharide, fuco-oligosaccharides, manno-oligosaccharides, xylo-oligosaccharides, sialyl-oligosaccharides, N-glycoprotein oligosaccharides, O-glycoprotein oligosaccharides, glycolipid oligosaccharides, cello-oligosaccharides, chitosan-oligosaccharides, chitin-oligosaccharides, galactourono-oligosaccharides, glucourono-oligosaccharides, beta-glucan oligosaccharides, arabinoxylo-oligosaccharides, arabinogalacto-oligosaccharides, xylogluco-oligosaccharides, galactomanno-oligosaccharides, rhamno-oligosaccharides, fructo[os]anes/inulins, galatans, fucoidans, arabinas, zylans, xanthans, beta-glucans, galacturonans, N-glycans, O-glycans, hyaluronic acids, chondroitins, xyloglucans, arabinogalactans, alginates, carageenanes, galactomannans, arabinoxylanes, glycolipid glycans, glycoprotein glycans, proteoglycans, reserve carbohydrates, fructans, galacto oligosaccharides from legumes, fucoidan, alpha-glucane, laminarin, carragenan, mannans, galactomannans, agar, natural gum, N-glycosidic bonded carbohydrates of glycoproteins, glycans of glycolipids, galacto-/gluco-/xylo-oligosaccharides, bacterial carbohydrates, xanthan, oligosaccharides: galacto-/gluco-(from alpha 1-2 and alpha 1-3 glucose residues), xylo-oligosaccharides, pectins, chitins; D-glucose: D-fructose; D-galactose; D-mannose; L-fucose: D-N-acetyl glucosamine; D-N-acetyl galactosamine; D-xylose: L-rhamnose; D-arabinose; D-allose; D-talose; L-idose; D-ribose; D-galacturon acid; D-glucuron; and D-mannuron acid; or
(D) a flavoring agent.

10. The nutritional supplement of claim 1, wherein the carbohydrate source includes at least one of the following: isomalt, isomaltulose, trehelose, D-tagatose, tapioca dextrin, and sucromalt.

11. The nutritional supplement of claim 1, wherein the supplement has a viscosity of less than about 120 centipoises; optionally wherein the supplement has a viscosity of less than about 100 centipoises or the supplement has a viscosity between about 50 centipoises and about 120 centipoises; or the supplement has a viscosity between about 50 centipoises and about 90 centipoises.

12. A method of producing a liquid nutritional supplement, the method comprising:
providing a mixture including water and a protein source including milk protein isolate;
subjecting the mixture to direct steam injection [DSI]; and
homogenizing the mixture,
wherein the liquid nutritional supplement has a viscosity of less than about 120 centipoises.

13. The method of claim 12, wherein
(A) the mixture further includes at least one of the following: an anti-foaming agent, a citrate, a fat source, a carbohydrate source, a vitamin, a mineral, and a flavouring agent; or
(B) the water has a temperature between about 120 and about 190 degrees Fahrenheit; optionally wherein the water has a temperature between about 125 and about 175 degrees Fahrenheit; optionally wherein the water has a temperature between about 130 and about 150 degrees Fahrenheit; optionally wherein the water has a temperature between about 135 and about 145 degrees Fahrenheit; or
(C) the DSI includes heating the mixture to about 250 degrees Fahrenheit for about 45 seconds; optionally wherein the DSI further includes flash cooling the mixture in a vacuum chamber to about 160 degrees Fahrenheit; or
(D) the method further comprises the step of:
(a) adding to the mixture at least one of the following: a citrate, a fat source, a carbohydrate source, a vitamin, a mineral, and a flavoring agent; or
(b) cooling the mixture to less than about 45 degrees Fahrenheit; or
(c) subjecting the mixture to a second DSI, wherein the second DSI includes heating the mixture to a temperature greater than about 280 degrees Fahrenheit.

14. A nutritional supplement selected from the group consisting of those of claims 1-34 for use in treating a nutritional deficiency in an individual.

15. The nutritional supplement of claim 14, wherein
(A) the treatment comprises administering to the individual at least one medicament; optionally wherein the nutritional supplement is administered enterally;
or
(B) the nutritional supplement is administered orally; or
(C) the individual is suffering from at least one of the following: anorexia nervosa; a wasting disease, including cancer, acquired immune deficiency syndrome (AIDS), and sarcopenia; chronic illness; functional limitations, including psychological disorders and physical incapacitation; diminished cognitive ability; and a wound.
